(19) Europäisches Patentamt
European Patent Office
Office européen des brevets

(11) **EP 2 219 581 B1**

(12) **EUROPEAN PATENT SPECIFICATION**

(45) Date of publication and mention
of the grant of the patent:
**19.03.2014   Bulletin 2014/12**

(21) Application number: **07835434.7**

(22) Date of filing: **15.11.2007**

(51) Int Cl.:
***A61F 15/00*** *(2006.01)*

(86) International application number:
**PCT/SE2007/050851**

(87) International publication number:
**WO 2009/064231 (22.05.2009 Gazette 2009/21)**

(54) **DISPENSER FOR ABSORBENT ARTICLES**

SPENDER FÜR SAUGFÄHIGE ARTIKEL

DISTRIBUTEUR POUR DES ARTICLES ABSORBANTS

(84) Designated Contracting States:
**AT BE BG CH CY CZ DE DK EE ES FI FR GB GR
HU IE IS IT LI LT LU LV MC MT NL PL PT RO SE
SI SK TR**

(43) Date of publication of application:
**25.08.2010   Bulletin 2010/34**

(73) Proprietor: **SCA Hygiene Products AB
405 03 Göteborg (SE)**

(72) Inventor: **JOHANSSON, Charlotte
S-417 27 Göteborg (SE)**

(74) Representative: **Valea AB
Lindholmspiren 5
417 56 Göteborg (SE)**

(56) References cited:
**WO-A1-2005/051261      WO-A1-2005/051261
US-A- 4 872 593          US-A- 4 872 593
US-B1- 6 299 018**

**Description**

TECHNICAL FIELD

The present invention relates to a dispenser for absorbent articles in the form of sanitary napkins, panty liners, interlabial products, tampons, baby diapers or incontinence protectors.

BACKGROUND OF THE INVENTION

In order to enable a user of sanitary napkins or the like to bring with her a daily supply of sanitary napkins in a handbag or the like, sanitary napkins are often packaged in individual packages. However, for several reasons, the need for a sanitary napkin may unexpectedly arise and there is therefore a need for having supplies of such articles available in public places such as in public toilets, in shopping centres or the like, in hospitals, in pubs and restaurants, in hotels, in airports, in aeroplanes, busses and trains, in offices, etc.

This need has to some extent been solved by dispensers for absorbent articles such as sanitary napkins. Such dispensers typically comprise a housing comprising a store of absorbent articles, said housing having an outlet through which the articles are dispensed.

WO 99/26576 discloses a dispenser for interlabial devices which is of simple construction. The interlabial devices are arranged as a continuous array of individually packaged devices joined together by frangible connections.

US-B 1-6,254, 582 discloses a supply of panty liner pads formed by a continuous longitudinal array of pad segments forming a strip. The strip has locally weakened zones to facilitate tearing off segments from the strip without the use of a cutting tool. The strip is wrapped upon itself into a roll so that pressure sensitive positioning adhesive strips are placed into contact with release surfaces formed on the strip, thereby protecting the adhesive prior to use without the need for release paper.

WO 2005/051261 describes a dispenser for absorbent articles from a roll. The dispenser comprises means for separating absorbent articles from a release material, and means for storing the portion of release material from which said absorbent articles have been separated.

It is known to provide sanitary napkins with garment-facing surfaces which have a high coefficient of friction, so as to improve the adherence of the napkins to the wearer's underwear. High-friction surfaces may be used alone or in combination with garment adhesive. An example of a sanitary napkin with a high-friction garment-facing surface is provided in EP 1 493 411. However, the present invention is not concerned with absorbent articles which themselves have integral high-friction surfaces.

To allow controlled dispensing of the articles, dispensers such as those described above comprise friction means which deliver an absorbent article from a stack or roll to the outlet. This means relies on frictional contact with the absorbent article to remove it from the stack or roll and to convey it towards the outlet.

US 4,872,593 A relates to dispensers for packaged bandages and the like, and particularly to dispensers of the type of which can be used to dispense single flat packaged units stored in a cartridge by use of a roller, with power supplied either manually or electrically.

One particular problem which can occur in dispensers such as those described above is that frictional contact between the friction means and the sanitary napkin to be dispensed is not sufficient, and the friction means does not grip the leading sanitary napkin. This is especially relevant when the sanitary napkins are supplied in a stack. As it is the weight of the remaining napkins in a vertical stack which presses the leading sanitary napkin against the friction means, there often comes a point at which there is insufficient frictional force between the friction means and the leading sanitary napkin. The dispenser then becomes blocked, or fails to deliver an absorbent article.

This problem is further compounded by the fact that many absorbent articles such as sanitary napkins are packaged in some way or have some sort of cover which has a low coefficient of friction with the surroundings. For instance, sanitary napkins which have garment adhesive on their garment-facing surface often have release papers which have been treated with e.g. silicone so that they can be released from the adhesive when required. The presence of the silicone coating on the release paper reduces the friction forces with the components of the dispenser.

SUMMARY OF THE INVENTION

The present invention addresses the above-mentioned problems, and thus provides a dispenser for dispensing an absorbent article, said absorbent article being arranged in a stack or a roll of absorbent article, in which at least a portion of each absorbent article is covered by a cover wrapper. The dispenser comprises a housing, said housing comprising an outlet through which the absorbent articles are dispensed; support means for supporting said stack or roll and friction means which is arranged to make contact with the cover wrapper of a leading absorbent article and deliver said leading absorbent article from said stack or roll towards the outlet. The friction means has a friction material comprising that surface of the friction means which is arranged to make contact with the cover wrapper. The friction material has a static coefficient of friction against the surface of the cover wrapper as measured by the method described herein of at least 0.4, preferably at least 0.6, most preferably at least 1.0.

Suitably, the dispenser is adapted to support absorbent articles arranged in a stack. The friction means may comprise a belt. The dispenser further comprises an electric motor, control unit and activator for said control unit, arranged such that activation of the activator by a user causes the electric motor to drive the friction means and dispense an absorbent article. There is an in-built time delay such that - after a user has activated the activator and a first absorbent article has been dispensed - a certain minimum time expires before a second absorbent article can be dispensed, such as e.g. at least 30 seconds, at least 60 seconds, at least 120 seconds, at least 300 seconds or at least 600 seconds. The activator may be selected from the group consisting of a push-button, a lever, a wheel, an IR sensor, a camera, a coin-operated device and a magnetic sensor.

The dispenser according to the invention may be arranged such that - after delivering the leading absorbent article from said stack or roll towards the outlet - the friction means reverses its direction of movement a short distance, e.g. between 5 - 15mm.

The absorbent article may be a sanitary napkin or panty liner, and is preferably a sanitary napkin.

*DEFINITIONS*

In relation to the present invention, the term "absorbent article" is used to describe an article which is worn in contact with the body, in the anogenital region, and used to absorb bodily exudates such as faeces, urine or menstrual blood.

The absorbent articles of the present invention are "disposable", i.e. they are not intended to be cleaned or reused, but are rather discarded after use.

The term "leading sanitary napkin" or "leading absorbent article" is used to describe that sanitary napkin or absorbent article which is next to be dispensed upon activation of the dispenser. The term "following sanitary napkin" or "following absorbent article" is used to describe that sanitary napkin or absorbent article which lies after the leading sanitary napkin or leading absorbent article in the dispenser.

BRIEF DESCRIPTION OF THE DRAWINGS

The invention will now be described with reference to the appended schematic Figures, in which

Figure 1 shows a cross-sectional view of the dispenser of the invention in which the absorbent articles are arranged in a stack;
Figure 2 shows a second embodiment of the dispenser of the invention in which the absorbent articles are arranged in a roll;
Figures 3a-3c illustrate the workings of the dispenser according to one embodiment;
Figure 4 shows an apparatus used for measuring the static friction coefficient of the friction materials of the invention.
Figure 5 shows an apparatus used for assessing the dispensing ability of the friction materials of the invention.

DETAILED DESCRIPTION OF PREFERRED EMBODIMENTS

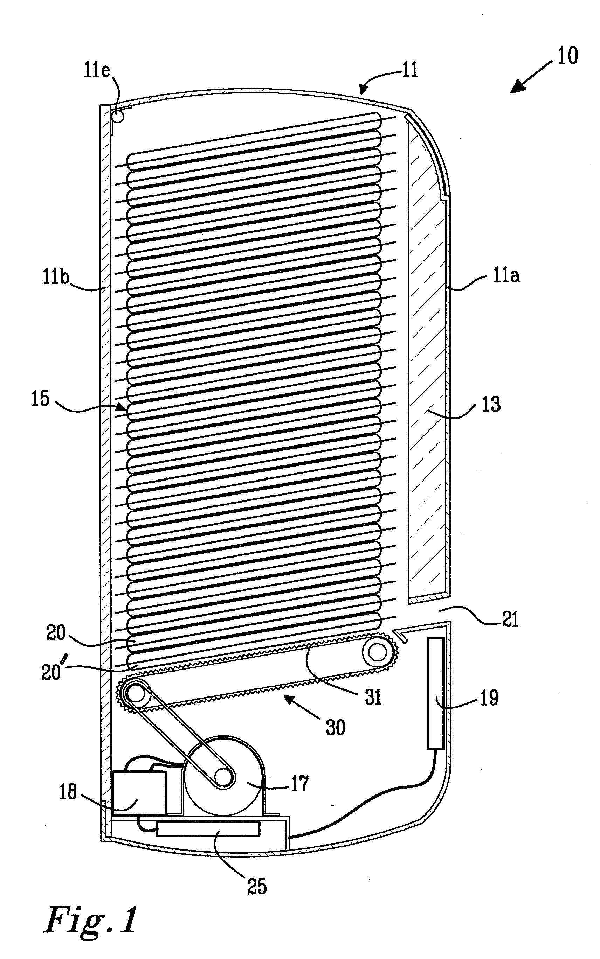

Figure 1 shows the dispenser 10 of the invention in cross-sectional view. The dispenser is designed to be placed on the wall or floor (free-standing) of a public places such as a public toilet. The dispenser 10 is designed for dispensing an individual absorbent article 20 from a stack 15 or roll 16 of such absorbent articles 20; a stack 15 in Figure 1.

The absorbent articles 20 to be dispensed by the dispenser 10 may be sanitary napkins, panty liners, interlabial products, tampons, baby diapers or incontinence protectors, of which sanitary napkins and panty liners are preferred. In the following, the invention is described in relation to a sanitary napkin, but is also applicable to the other absorbent articles listed above. Typically, such absorbent articles 20 comprise a liquid-permeable topsheet, a liquid impermeable backsheet and an absorbent body located between said topsheet and said backsheet. The absorbent article 20 may also comprise one or more layers which are designed to improve the handling of bodily waste e.g. transfer, distribution, surge or acquisition layers, which are usually located between the topsheet and the absorbent core.

The dispenser 10 comprises a housing 11. The housing may be formed in one or more sections, such as a front 11 a and rear section 11 b as shown in Figure 1. The housing 11 can be opened in some way so that the stack 15 or roll 16 of absorbent articles 20 can be easily introduced when the dispenser is to be refilled. For example, in the embodiment illustrated in Figure 1, the front section 11 a of the housing is connected to the rear section 11b via a hinge 11c and can be opened up to allow the dispenser 10 to be filled. Alternatively, a door may be present in the housing to allow the dispenser 10 to be filled with a stack 15 or roll 16 of absorbent articles 20. If the dispenser 10 is to be mounted on a wall, suitable wall-mounting means (e.g. holes for screws) can be included in the housing 11. The housing 11 is made of any suitable material which protects the contents of the dispenser 10 from e.g. dirt, damage or water. Metal or plastic are suitable materials for the dispenser housing 11. To prevent theft of absorbent articles 20, the housing 11 may be lockable with a key.

The housing 11 comprises an outlet 12, through which absorbent articles 20 are dispensed. The outlet 12 shown in Figure 1 comprises a narrow slit which is large enough for a single absorbent article 20 to pass through, but preferably small enough to prevent two or more absorbent articles 20 being dispensed, and preferably not large enough for a user to insert their hand into the dispenser 10.

The stack 15 or roll 16 of absorbent articles 20 are supported within the dispenser 10 on support means 13. In the case of the stack 15 of articles shown in Figure 1, the support means 13 comprises a channel on the inside of the housing 11 in which the stack 15 of absorbent articles rests. The support means 13 may be integral with the housing 11 (e.g. it may be formed in one piece with the housing 11) or it may comprise a separate component of the dispenser. In the case of a roll 16 of absorbent articles, a suitable support means 13 may be a spindle or roll-holder which supports the roll 16 at its centre, optionally via a roll core.

The stack 15 or roll 16 of absorbent articles can be considered as comprising a leading absorbent article 20', a following absorbent article, and further absorbent articles. As described above, the term "leading absorbent article" is used to describe the absorbent article which is next to be dispensed upon activation of the dispenser (e.g. that lying at the top or bottom of a stack 15, or outermost on a roll 16).

At least a portion of each absorbent article 20 in a stack 15 or roll 16 is covered by a cover wrapper 22. Preferably, the entirety of each absorbent article 20 is covered by a cover wrapper 22, as shown in Figure 1. The cover wrapper 22 is used to protect the absorbent article 20 from the surroundings, e.g. dirt, liquids or physical damage, and is removed before use of the absorbent article 20. Suitably, the cover wrapper 22 comprises a plastic film of e.g. polyethylene, polypropylene or polyester, or a laminate of one or more films. The cover wrapper 22 may comprise materials such as e.g. plastic films paper, nonwoven materials, metal foils or combinations or laminates thereof. The cover wrapper 22 is typically the same on all sides of the absorbent article 20; however, it is conceivable that an absorbent article 20 is covered on one side by one cover wrapper material and on another side by a different cover wrapper material.

In addition to the above-mentioned components, the housing 11 of the dispenser comprises friction means 30 which is arranged to make contact with the cover wrapper 22 of a leading absorbent article 20'. The leading absorbent article 20' may be supported on the friction means 30, as shown in Figure 1. Alternatively, the leading absorbent article may be held against the friction means 30 by means of a sprung member or a roller. The friction means 30 delivers said leading absorbent article 20' from said stack 15 or roll 16 towards the outlet 12 of the dispenser housing 11, and typically takes the form of a roller or belt. Figure 1 illustrates the friction means 30 as a belt spanning two supporting rollers. The upper surface of the belt contacts the leading absorbent article 20'.

Additional means for delivering the absorbent article 20 towards the outlet 12 may be present between the friction means 30 and the outlet 12. For instance, the absorbent article may have to pass along one or more chutes or channels or over one or more rollers or belts before being dispensed from the dispenser 10 through the outlet 12.

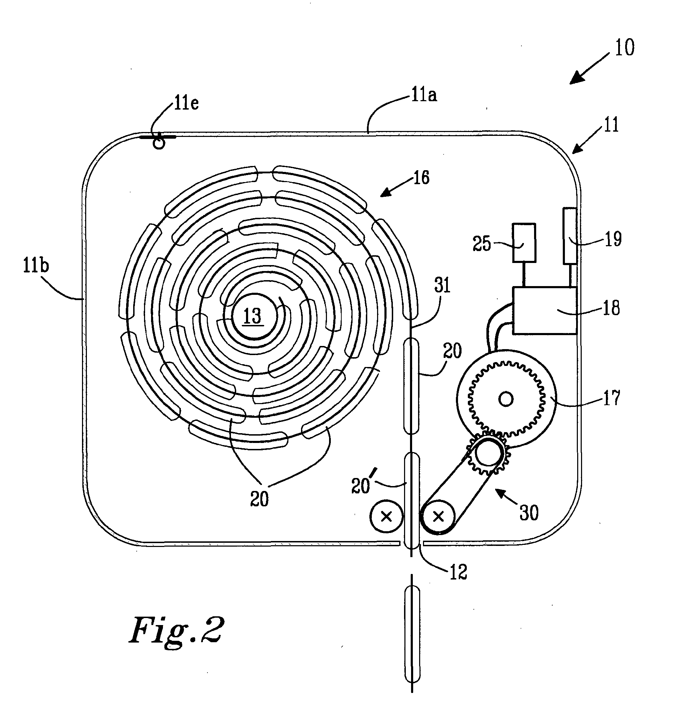

The surface of the friction means 30 which makes contact with the cover wrapper 22 of the leading absorbent article 20' comprises a friction material 31. The friction material 31 may be integral with the friction means 30 (i.e. it may comprise the outer surface of the friction means 30) or it may comprise a separate component. For example, if the friction means 30 is a cylinder or wheel, the friction material 31 may be the outer surface of said cylinder or wheel, or it may be a sleeve located on the cylinder or wheel. Figures 1 and 2 show the friction means 30 as a belt supported by two rollers, in which the material of the belt comprises the friction material 31.

The surface of the cover wrapper 22 which contacts the friction means 30 has a static coefficient of friction against the friction material 31 as measured by the method described herein of at least 0.4, preferably at least 0.6, most preferably at least 1.0.

Friction forces are categorized as either static or kinetic. Static friction is generally understood as the force required to start an object moving against another, while kinetic friction is the force required to maintain movement, once initiated. The present invention relates solely to static friction.

The coefficient of friction between two surfaces relates the frictional force exerted on an object (e.g. that required to initiate movement) to the normal force exerted between the two surfaces (e.g. the weight of an object resting on a surface). The coefficient of static friction is typically greater than that of kinetic friction.

The static friction force must be overcome by an applied force before an object will move. Multiplying the coefficient of static friction by the normal force provides the maximum possible friction force between two surfaces before sliding begins: $F_{max} = \mu_s N$. Before any sliding occurs, the friction force takes any value from zero up to $F_{max}$. Any force smaller than $F_{max}$ attempting to slide one surface over the other will be opposed by a frictional force of equal magnitude and opposite direction. Any force larger than $F_{max}$, will overcome the force of static friction and cause sliding to occur. When sliding occurs, kinetic friction is applicable, and static friction is no longer relevant.

A dispenser 10 in which the surface of the cover wrapper 22 which contacts the friction means 30 has the minimum static coefficient of friction against the friction material 31 set out above addresses the problems associated with the prior art. In particular, the tendency of the friction material 31 to slip against the cover wrapper 22 is reduced. In this way, effective and reliable dispensing of absorbent articles 20 can be obtained, as the number of times a dispenser 10 fails to dispense an absorbent article 20 through slippage of the two contact surfaces is reduced.

Figure 2 illustrates the dispenser 10 of the invention arranged for dispensing absorbent articles 20 (also sanitary napkins in this case) in the form of a roll 16. Absorbent articles 20 are connected in a row via their cover wrappers 22, and the row of absorbent articles 20 is wound to form a roll 16. Weakened points or perforations in the cover wrapper 22 between adjacent absorbent articles 20 allow the leading absorbent article 20' to be removed from the roll 16.

Problems relating to friction between the cover wrapper 22 of the sanitary napkin 20 and the friction means 30 / friction material 31 also arise in the case of a roll 16. In order for a sanitary napkin 20 to be dispensed, the roll 16 must be set in rotary motion, and its rotational inertia must therefore be overcome. When a roll 16 is new, and contains many sanitary napkins 20, it has relatively high rotational inertia. The present invention has realised - in such cases - that a sufficiently high static coefficient of friction must be present between the leading sanitary napkin 20' and the friction material 31 for effective dispensing.

The dispenser 10 according to the invention may be operated in a number of ways. The dispenser may be manually operated, so that e.g. a lever, push-button or wheel is used to drive the friction means 30 mechanically.

The dispenser 10 is operated electrically, and therefore comprises an electric motor 17, a control unit 18 and activator 19 for said control unit 18. These components are arranged such that activation of the activator 19 by a user causes the electric motor 17 to drive the friction means 30 and dispense an absorbent article 20. A suitable activator 19 for the control unit 18 may be a push-button, a lever, a wheel, an IR sensor, a camera, or a magnetic sensor. The activator 19 may also be a coin-operated device.

The control unit 18 comprises the necessary circuitry to receive a signal from the activator 19 and to send a signal to the electric motor 17. The control unit 18 introduces an in-built time delay, so that - after a user has activated the activator 19 and a first absorbent article 20 has been dispensed - a certain minimum time expires before a second absorbent article can be dispensed. The time delay may be e.g. at least 30 seconds, at least 60 seconds, at least 120 seconds, at least 300 seconds or at least 600 seconds. This time delay discourages the theft of large numbers of sanitary napkins 20 from the dispenser 10, as a potential thief will have to wait a long time to be able to collect more than a few absorbent articles 20.

An electrically-operated dispenser 10 requires a power source, which is illustrated as batteries 25 in Figures 1 and 2. However, other sources of power are possible, such as e.g. mains electricity or solar power.

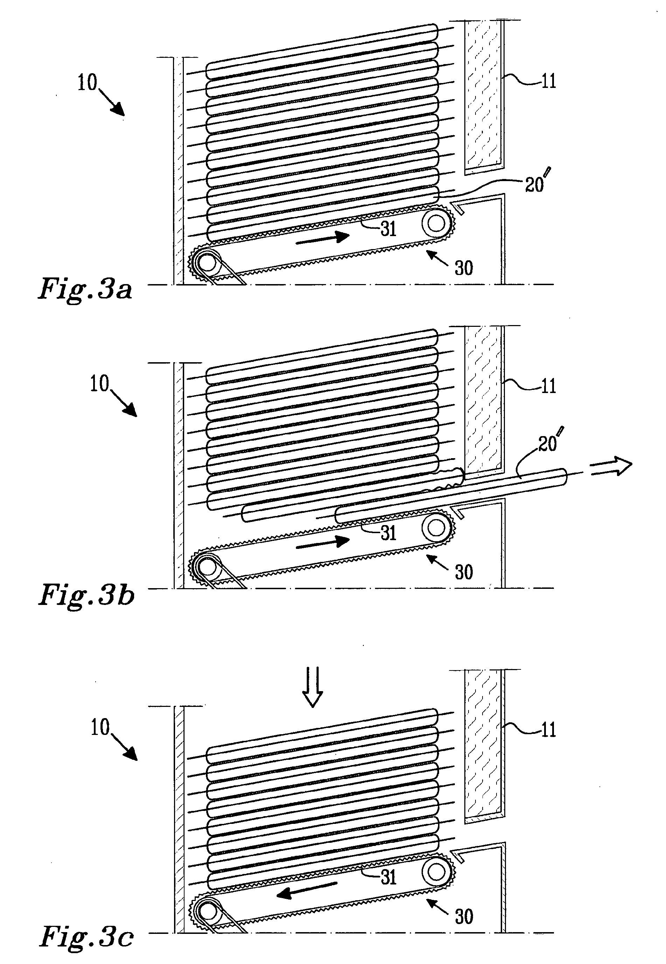

It is also advantageous that the dispenser 10 according to the invention is arranged such that - after delivering said leading absorbent article 20' from said stack 15 or roll 16 towards the outlet 12 - the friction means 30 reverses its direction of movement. This is illustrated in Figures 3a - 3c. This is an advantage, as a following absorbent article will often follow the leading absorbent article 20' a certain distance when the leading absorbent article 20' is dispensed. In the case of absorbent articles 20 arranged in a stack 15, the following absorbent article may therefore be pressed against the housing 11 of the dispenser 10, and thereby suffer damage or exert a residual force on the friction means 30 (Figure 3b). By reversing the direction of movement of the friction means, the following absorbent article is returned to its correct place in the stack 15 (Figure 3c). The reverse movement is carried out for a short distance, e.g. between 5 - 15mm. In the case of a roll 16 of absorbent articles 20, reversing the direction of movement of the friction means a short distance prevents overshoot of the roll 16.

DESCRIPTION OF TEST METHODS

*Measurement of Static Coefficient of Friction*

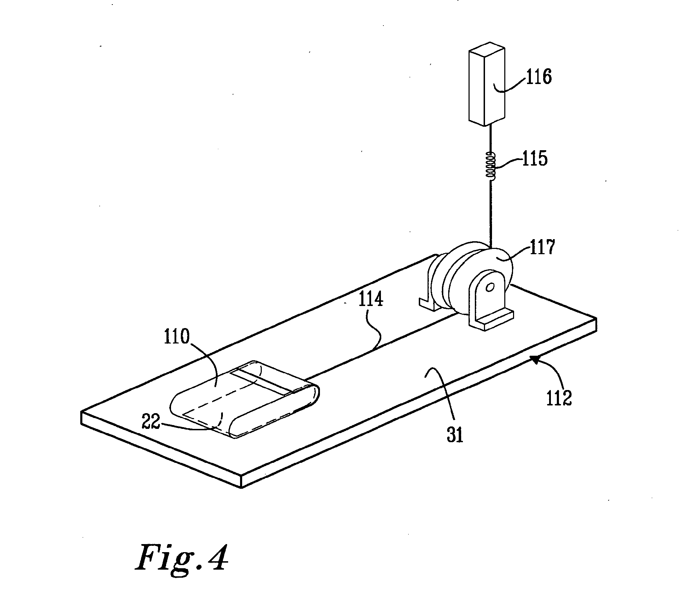

The static coefficient of friction between two materials is measured according to DIN test method 53 375 (November 1996). A simplified diagram of the apparatus used in the test method is shown in Figure 4.

The apparatus consists of a sledge 110 which is adapted to be pulled over a horizontal table 112 by means of an inelastic nylon cord 114. The sledge 110 is 64mm wide, 63mm long and has a mass of 200g. A tensile tester 116 is attached to the remote end of the nylon cord 114, via a spring 115. The tensile tester 116 used in these experiments is an Instron 4301 apparatus, with a static load cell of maximum load 10N, connected to a computer for recording, calculating and storing the results. The spring 115 was obtained from Stock Spring in Lesjöfors, Sweden (Art. No. 3285) and has a force constant of 0.204N/mm. In this particular case, the nylon cord 114 runs through a pulley 117 to change the direction of action.

A sample of cover wrapper 22 having the same width as the sledge 110 is placed on the underside of the sledge 110, so that the entire lower surface of the sledge 110 is covered by cover wrapper material. The cover wrapper 22 is folded over the leading edge of the sledge 110 in the direction of travel, and secured to the upper surface of the sledge 110 with double-sided sticky tape, allowing the back part to hang free. The sledge 110 and cover wrapper 22 are conditioned for 4h at $50 \pm 5\%$ RH and $23 \pm 1$ °C.

The table 112 consists of the friction material 31 defined in the present invention; i.e. that surface of the friction means 30 which makes contact with the cover wrapper 22 in the dispenser 10. A sample of the friction material 31 with dimensions corresponding to those of the table 112 is flattened out and fastened with double-sided tape to the table 112.

The sledge 110 and cover wrapper 22 are placed on the friction material 31, with the nylon cord 114 directed towards the pulley 117, such that the sample of cover wrapper 22 lies flat. The tensile tester 116 is zeroed with the sledge 110 lying on the friction material 31, without load. The cord 114 is tightened to 0.05N, and the tensile tester is zeroed again. The tensile tester 116 is started, and begins pulling on the nylon cord 114, via spring 115. The tensile tester 116 records the force required to start movement of the sledge 110.

After each test, a new cover wrapper 22 and friction material 31 were used. Five tests were carried out for each friction material. The results are presented by the apparatus as a chart, with time on the x-axis and force on the y-axis. The peak y-value of the curve is used to calculate the coefficient of static friction.

The static coefficient of friction $\mu_s$ is calculated as follows:

$$\mu_s = \frac{F_S}{F_N}$$

wherein $F_s$ = the force require to overcome adhesive friction. $F_s$ is calculated by:

$$F_S = F - \Delta$$

in which F is the force in Newtons obtained during the tests and $\Delta$ is calculated by:

$$\Delta = \frac{v \bullet \sqrt{D/m}}{g}$$

in which $v$ is the pulling speed (= 100mm/60s), D is the spring constant of the spring (= 0.204N/m $\approx$ 2.105 g/s$^2$), m is the mass of the sledge (= 200g) and g is the gravitational acceleration constant (9.81 m/s$^2$). $\Delta$ is a measure of the inertia of the sledge, which produces an inertia force at the beginning of the sledge's movement. The value of the adhesive friction $F_s$ is therefore changed by the small amount $\Delta$.

Furthermore $F_N$ is obtained by;

$$F_N = w \times g$$

wherein w is the mass of the sledge (= 200g) and g is the gravitational acceleration constant (9.81 m/s$^2$).

In the following experiments, the cover wrapper material was a 30gsm blown polyethylene film which is commercially available from M&W Verpackungen, supplier code KC 5080.010.

Eight different samples of friction material were used, which are shown in Table 1 (Figure 6). All friction materials were obtained from Chiorino S.p.A, Biella, Italy, apart from sample 4 which was obtained from Sampla Belting S.p.A., Italy.

| | | | Conveying side | | Complete Belt | | |
|---|---|---|---|---|---|---|---|
| Sample | Supplier Code | Material | Thickness | Thickness | Elongation at 1% | Weight |
| 1 | NA-136 | Synthetic elastomer | 0.2 mm | 1.2 mm | 3 N/mm | 1.2 kg/m$^2$ |
| 2 | NA-842 | Polyurethane (TPU) | 0.2 mm | 1.4 mm | 6 N/mm | 1.4 kg/m$^2$ |
| 3 | NA-49 | Polyvinyl chloride (PVC) | 0.3 mm | 1.8 mm | 6 N/mm | 2.0 kg/m$^2$ |
| 4 | 5X92 | Polyvinyl chloride (PVC) | 0.4 mm | 1.8 mm | -- | 2.1 kg/m$^2$ |
| 5 | NA-30 | Polyvinyl chloride (PVC) | 0.5 mm | 2.0 mm | 8 N/mm | 2.3 kg/m$^2$ |
| 6 | NA-128 | Silicone | 0.2 mm | 1.4 mm | 12 N/mm | 1.3 kg/m$^2$ |

(continued)

| Sample | Supplier Code | Conveying side | | Complete Belt | | |
| | | Material | Thickness | Thickness | Elongation at 1% | Weight |
|---|---|---|---|---|---|---|
| 7 | NA-716 | Fabric with polyurethane (TPU) impregnation | -- | 1.0 mm | 6 N/mm | 1.0 kg/m$^2$ |
| 8 | NA-135 | Fabric with polyurethane (TPU) impregnation | -- | 1.0 mm | 3 N/mm | 0.9 kg/m$^2$ |

Table 2 shows the static coefficient of friction obtained for friction materials 1-8 using the method described above.

**Table 2**

| Sample | Load at first peak (N), average of 5 tests | Static coefficient of friction |
|---|---|---|
| 1 | 2.98 | 1.51 |
| 2 | 1.28 | 0.64 |
| 3 | 2.44 | 1.23 |
| 4 | 2.25 | 1.14 |
| 5 | 2.02 | 1.02 |
| 6 | 0.94 | 0.48 |
| 7 | 0.65 | 0.33 |
| 8 | 0.73 | 0.37 |

*Assessment of dispensing ability of friction materials*

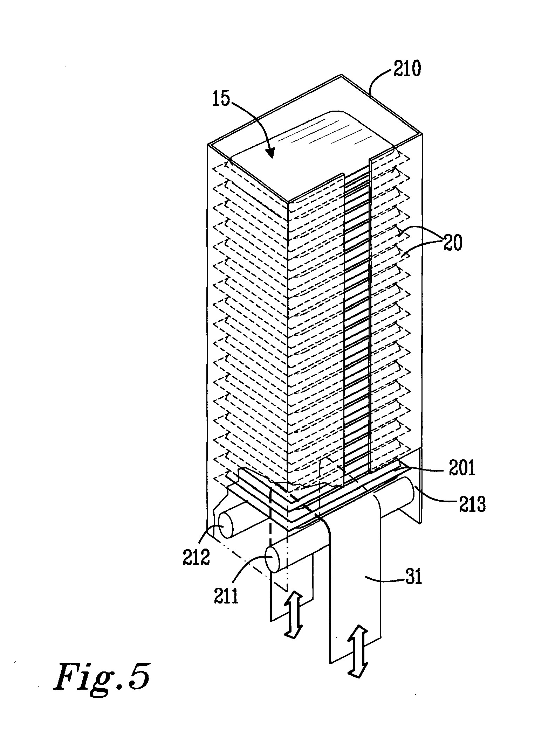

The ability of the friction materials 1-8 to dispense sanitary napkins from a stack was modelled using the apparatus shown in Figure 5.

The apparatus shown in Figure 5 comprises a housing 210 for a stack 15 of sanitary napkins 20, which has a square cross-section with an edge-length of 74mm. The sanitary napkins 20 are commercially available under the name Libresse Nana Normal, which in their individual packaged form in the above-described cover wrapper are tri-folded to have dimensions of circa 68 mm by 74mm and a weight of 3.27g.

A front 211 spindle and a rear spindle 212 are arranged in parallel at the bottom of the housing 210, such that they extend laterally across the housing 210, as shown in Figure 5. The distance between the outer (distal) edges of the two spindles 211, 212 is 70mm.

The front of the housing 210 is cut away in front of the front spindle 211 in a direction parallel to the extension of the spindles 211, 212, to provide an outlet 213 for the leading sanitary napkin 20'. The outlet 213 in this apparatus extends the entire width of the housing, and extends to a distance of approximately 13mm above the surface defined by the upper edges of the spindles 211, 212.

A strip of friction material 31 is provided having a width of 34mm, and a length which is at least long enough to pass over both spindles and hang down at the front and rear of the apparatus and allow both gripping and 25mm of movement during dispensing as described below. The strip is passed over the front and rear spindles 211, 212 as shown in Figure 5, such that the ends hang down at the front and rear of the housing 210.

A stack of 35 sanitary napkins is placed within the housing 210, so that the leading sanitary napkin 20' makes contact with and is supported by the friction material 31, as shown in Figure 5. The contact area between the leading sanitary napkin 20' and the friction material is ca. 1700mm$^2$.

The strip of friction material 31 is pulled tightly across the spindles 211, 212, and - while maintaining the tension in the friction material 31 - the front and rear ends of the friction material 31 are pulled alternately, so as to move the friction material 31 through the housing 210. The friction material 31 moves enough to dispense a single sanitary napkin 20 (ca. 25mm), and is then returned to its original position.

When moving forwards, the friction material 31 has a static coefficient of friction with the cover wrapper 22 of the leading sanitary napkin 20' which is sufficient to drag the leading sanitary napkin 20' from under the stack 15 and deliver it through the outlet 213.

The friction material 31 is moved back and forth repeatedly until all sanitary napkins 20 are dispensed from the housing 210. If a sanitary napkin 20 fails to be dispensed upon pulling the friction material forward (e.g. it does not grip the friction material 31) a "fail" is recorded. The "failed" napkin is removed manually and the test continued.

Each test is repeated five times for each friction material 31. The results are shown in Table 3.

**Table 3:**

| Friction Material | Test no. | Total no. of products dispensed | Which product(s) failed | Comments |
|---|---|---|---|---|
| 1 | 1 | 35 | | |
| | 2 | 34 | 35 | |
| | 3 | 35 | | |
| | 4 | 34 | 34 | |
| | 5 | 35 | | |
| 2 | 1 | 35 | | |
| | 2 | 34 | 34 | |
| | 3 | 34 | 35 | |
| | 4 | 34 | 33 | |
| | 5 | 34 | 34 | |
| 3 | 1 | 34 | 35 | |
| | 2 | 34 | 35 | |
| | 3 | 33 | 34,35 | |
| | 4 | 33 | 31,34 | |
| | 5 | 34 | 31 | |
| 4 | 1 | 34 | 34 | 22 caught at the edge |
| | 2 | 34 | 35 | 28 caught at the edge |
| | 3 | 34 | 33 | |
| | 4 | 35 | | |
| | 5 | 34 | 34 | |
| 5 | 1 | 34 | 35 | |
| | 2 | 35 | | |
| | 3 | 33 | 24,34 | 31 caught at the edge |
| | 4 | 35 | | |
| | 5 | 34 | 34 | |
| 6 | 1 | 35 | | |
| | 2 | 33 | 33,35 | 31 caught at the edge |
| | 3 | 32 | 5,28,35 | |
| | 4 | 29 | 12,16,17,31,32,35 | The material gathered dirt and gave worse results upon repeating |
| | 5 | 28 | 16,23,27,26,28,32,35 | |
| 7 | 1 | <5 | Friction of material is too low and only between ca. 2 and 5 products were dispensed each time | |
| | 2 | <5 | | |
| | 3 | <5 | | |
| | 4 | <5 | | |
| | 5 | <5 | | |
| 8 | 1 | <5 | Friction of material is too low and only between ca. 2 and 5 products were dispensed each time | |
| | 2 | <5 | | |
| | 3 | <5 | | |
| | 4 | <5 | | |
| | 5 | <5 | | |

The friction materials 7 and 8 were not suitable as a friction material for the dispenser, as they failed to dispense a number of products. Other materials were suitable friction materials for the dispenser. Material 6 gripped products well, but had the tendency to gather dirt. Materials 1-5 generally functioned well. It is noticeable that problems often occurred in dispensing the last absorbent article, as there was no stack pressure acting upon it.

The invention has been described with reference to a number of embodiments. However, the scope of the invention should be defined by the appended claims. In particular, the dispenser of the invention may also be used to dispense other absorbent articles such as panty liners, interlabial products, tampons, baby diapers or incontinence protectors.

**Claims**

1. A dispenser (10) comprising absorbent articles (20) in a stack (15) or a roll (16), in which at least a portion of each absorbent article (20) is covered by a cover wrapper (22);
said dispenser (10) comprising a housing (11), said housing (11) comprising:

an outlet (12) through which the absorbent articles (20) are dispensed;
support means (13) for supporting said stack (15) or roll (16);
and friction means (30) which is arranged to make contact with the cover wrapper (22) of a leading absorbent article (20') and deliver said leading absorbent article (20') from said stack (15) or roll (16) towards the outlet (12);
said friction means (30) having a friction material (31) comprising that surface of the friction means (30) which is arranged to make contact with the cover wrapper (22);
wherein the cover wrapper (22) is a plastic film or a laminate of one or more films, and wherein
the friction material (31) has a static coefficient of friction against the surface of the cover wrapper (22) as measured by the method described herein of at least 0.4, preferably at least 0.6, most preferably at least 1.0
wherein said dispenser (10) further comprises an electric motor (17), control unit (18) and activator (19) for said control unit (18), arranged such that activation of the activator (19) by a user causes the electric motor (17) to drive the friction means (30) and dispense an absorbent article (20) *characterised in that* the dispenser (10) has is an in-built time delay such that - after a user has activated the activator (19) and a first absorbent article (20) has been dispensed - a certain minimum time expires before a second absorbent article (20) can be dispensed, such as e.g. at least 30 seconds, at least 60 seconds, at least 120 seconds, at least 300 seconds or at least 600 seconds.

2. A dispenser according to claim 1, wherein the dispenser (10) is adapted to support absorbent articles (20) being arranged in a stack (15).

3. A dispenser (10) according to any of the preceding claims, wherein the friction means (30) comprises a belt.

4. A dispenser (10) according to any of the preceding claims, wherein the activator (19) is selected from the group consisting of a push-button, a lever, a wheel, an IR sensor, a camera, a coin-operated device and a magnetic sensor.

5. A dispenser (10) according to any of the preceding claims, arranged such that - after delivering said leading absorbent article (20') from said stack (15) or roll (16) towards the outlet (12) - the friction means (30) reverses its direction of movement a short distance, e.g. between 5 -15mm.

6. A dispenser (10) according to any of the preceding claims, wherein the absorbent article (20) is a sanitary napkin or panty liner, preferably a sanitary napkin.

**Patentansprüche**

1. Spender (10) mit saugfähigen Artikeln (20) in einem Stapel (15) oder einer Rolle (16), in dem bzw. in der zumindest ein Abschnitt von jedem saugfähigen Artikel (20) durch einen Abdeckumschlag (22) bedeckt ist;
wobei der Spender (10) ein Gehäuse (11) aufweist, das Gehäuse (11) mit:

einem Auslass (12), durch den die saugfähigen Artikel (20) ausgegeben werden;
einem Stützmittel (13) zum Unterstützen des Stapels (15) oder der Rolle (16); und
einem Reibmittel (30), das angeordnet ist, den Abdeckumschlag (22) eines führenden saugfähigen Artikels (20') in Kontakt zu bringen und den führenden saugfähigen Artikel (20') von dem Stapel (15) oder der Rolle (16) in Richtung des Auslasses (12) zu führen;

wobei das Reibmittel (30) ein Reibmaterial (31) aufweist, das die Oberfläche des Reibmittels (30) aufweist und angeordnet ist, um den Abdeckumschlag (22) in Kontakt zu bringen;

der Abdeckumschlag (22) eine Plastikfolie oder ein Laminat aus einer oder mehreren Folien ist,

das Reibmaterial (31) einen Haftreibwert gegenüber der Oberfläche des Abdeckumschlags (22), gemessen durch die hierin beschriebene Methode, von mindestens 0,4, bevorzugt von mindestens 0,6, am bevorzugtesten von mindestens 1,0 aufweist, und

der Spender (10) ferner einen Elektromotor (17), eine Steuereinheit (18) und einen Aktivator (19) für die Steuereinheit (18) aufweist, die so angeordnet sind, dass eine Aktivierung des Aktivators (19) durch einen Benutzer verursacht, dass der Elektromotor (17) das Reibmittel (30) antreibt und einen saugfähigen Artikel (20) ausgibt, **dadurch gekennzeichnet, dass**

der Spender (10) eine eingebaute Zeitverzögerung aufweist, so dass, nachdem ein Benutzer den Aktivator (19) aktiviert hat und ein erster saugfähiger Artikel (20) abgegeben worden ist, eine bestimmte minimale Zeitspanne vergeht, bevor ein zweiter saugfähiger Artikel (20) abgegeben werden kann, wie zum Beispiel mindestens 30 Sekunden, mindestens 60 Sekunden, mindestens 120 Sekunden, mindestens 300 Sekunden oder mindestens 600 Sekunden.

2. Spender nach Anspruch 1, wobei der Spender (10) angepasst ist, die in einem Stapel (15) angeordneten saugfähigen Artikel (20) zu unterstützen.

3. Spender (10) nach einem der vorstehenden Ansprüche, bei dem das Reibmittel (30) ein Band aufweist.

4. Spender (10) nach einem der vorstehenden Ansprüche, bei dem der Aktivator (19) aus der Gruppe ausgewählt ist, die aus einem Druckknopf, einem Hebel, einem Rad, einem IR-Sensor, einer Kamera, einer münzbetätigten Einrichtung und einem Magnetsensor besteht.

5. Spender (10) nach einem der vorstehenden Ansprüche, der so angeordnet ist, dass nachdem der führende saugfähige Artikel (20') von dem Stapel (15) oder der Rolle (16) in Richtung des Auslasses (12) ausgegeben worden ist, das Reibmittel (30) seine Bewegungsrichtung über eine kurze Distanz umdreht, zum Beispiel zwischen 5 - 15 mm.

6. Spender (10) nach einem der vorstehenden Ansprüche, bei dem der saugfähige Artikel (20) eine Damenbinde oder ein Pantyliner, bevorzugt eine Damenbinde, ist.

**Revendications**

1. Distributeur (10) comprenant des articles absorbants (20) disposés en pile (15) ou en rouleau (16), dans lequel au moins une partie de chaque article absorbant (20) est recouverte d'une enveloppe de protection (22) ; ledit distributeur (10) comprenant un boîtier (11), ledit boîtier (11) comprenant :

une ouverture de sortie (12) par laquelle les articles absorbants (20) sont distribués ;

un moyen de support (13) pour supporter ladite pile (15) ou ledit rouleau (16) ;

et un moyen de friction (30) qui est adapté pour établir un contact avec l'enveloppe de protection (22) d'un article absorbant de tête (20') et transférer ledit article absorbant de tête (20') de ladite pile (15) ou dudit rouleau (16) vers l'ouverture de sortie (12) ;

ledit moyen de friction (30) comportant un matériau de friction (31) qui constitue la surface du moyen de friction (30) qui est adaptée pour établir un contact avec l'enveloppe de protection (22) ;

dans lequel l'enveloppe de protection (22) est un film en plastique ou une superposition d'un ou de plusieurs films, et dans lequel

le matériau de friction (31) a un coefficient de frottement statique contre la surface de l'enveloppe de protection (22), tel que mesuré par le procédé décrit dans la présente, d'au moins 0,4, de préférence d'au moins 0,6 et mieux encore d'au moins 1,0 ;

dans lequel ledit distributeur (10) comprend en outre un moteur électrique (17), une unité de commande (18) et un élément de déclenchement (19) pour ladite unité de commande (18), agencés de telle manière que l'actionnement de l'élément de déclenchement (19) par un utilisateur fait que le moteur électrique (17) entraîne le moyen de friction (30) et délivre un article absorbant (20),

**caractérisé en ce que** le distributeur (10) a un circuit temporisé incorporé tel que, après qu'un utilisateur a actionné l'élément de déclenchement (19) et qu'un premier article absorbant (20) a été distribué, un certain laps de temps minimum s'écoule avant qu'un deuxième article absorbant (20) puisse être distribué, comme par exemple au moins 30 secondes, au moins 60 secondes, au moins 120 secondes, au moins 300 secondes ou au moins 600 secondes.

2. Distributeur selon la revendication 1, dans lequel le distributeur (10) est adapté pour supporter des articles absorbants (20) disposés en pile (15).

3. Distributeur (10) selon l'une quelconque des revendications précédentes, dans lequel le moyen de friction (30) comprend une courroie.

4. Distributeur (10) selon l'une quelconque des revendications précédentes, dans lequel l'élément de déclenchement (19) est choisi dans le groupe comprenant un bouton poussoir, un levier, une roue, un capteur infrarouge, une caméra, un dispositif à pièces de monnaie et un capteur magnétique.

5. Distributeur (10) selon l'une quelconque des revendications précédentes, agencé de telle manière que, après la distribution dudit article absorbant de tête (20') de ladite pile (15) ou dudit rouleau (16) vers l'ouverture de sortie (12), le moyen de friction (30) inverse sa direction de déplacement sur une courte distance, par exemple égale de 5 à 15 mm.

6. Distributeur (10) selon l'une quelconque des revendications précédentes, dans lequel l'article absorbant (20) est une serviette hygiénique ou un protège-slip, de préférence une serviette hygiénique.

*Fig.1*

*Fig.2*

Fig.3a

Fig.3b

Fig.3c

*Fig.4*

*Fig.5*

**EP 2 219 581 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9926576 A
- US 6254582 B1
- WO 2005051261 A
- EP 1493411 A
- US 4872593 A